(19) Europäisches Patentamt
European Patent Office
Office européen des brevets

(11) **EP 3 495 821 B1**

(12) **EUROPEAN PATENT SPECIFICATION**

(45) Date of publication and mention
of the grant of the patent:
**17.02.2021 Bulletin 2021/07**

(51) Int Cl.:
***G01N 33/573*** (2006.01)     ***C12Q 1/26*** (2006.01)
***G01N 33/577*** (2006.01)

(21) Application number: **18210247.5**

(22) Date of filing: **04.12.2018**

(54) **DIAGNOSTIC METHOD FOR DETERMINING NOX2 PROTEIN**

DIAGNOSTISCHES VERFAHREN ZUR BESTIMMUNG DES NOX2-PROTEINS

PROCÉDÉ DE DIAGNOSTIC PERMETTANT DE DÉTERMINER LA PROTÉINE NOX2

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **07.12.2017 IT 201700141849**

(43) Date of publication of application:
**12.06.2019 Bulletin 2019/24**

(73) Proprietor: **Università degli Studi di Roma "La
Sapienza"**
**00185 Roma (IT)**

(72) Inventors:
• **VIOLI, Mr. Francesco**
**I-00199 Roma (IT)**
• **PIGNATELLI, Mr. Pasquale**
**I-00199 Roma (IT)**
• **CARNEVALE, Mr. Roberto**
**I-04022 Fondi (LT) (IT)**

(74) Representative: **Freyria Fava, Cristina
Buzzi, Notaro & Antonielli d'Oulx S.p.A.
Corso Vittorio Emanuele II, 6
10123 Torino (IT)**

(56) References cited:
**US-A1- 2012 156 704**

• **PACLET MARIE-HELENE ET AL: "Localization of
Nox2 N-terminus using polyclonal antipeptide
antibodies", BIOCHEMICAL JOURNAL,
PORTLAND PRESS LTD, GB, vol. 382, no. Part 3,
15 September 2004 (2004-09-15), pages 981-986,
XP002603667, ISSN: 0264-6021**
• **CAMPION ET AL: "New insights into the
membrane topology of the phagocyte NADPH
oxidase: Characterization of an anti-gp91-phox
conformational monoclonal antibody",
BIOCHIMIE, MASSON, PARIS, FR, vol. 89, no. 9,
17 August 2007 (2007-08-17), pages 1145-1158,
XP022206546, ISSN: 0300-9084, DOI:
10.1016/J.BIOCHI.2007.01.010**
• **SEAN J COONEY ET AL: "Cellular and temporal
expression of NADPH oxidase (NOX) isotypes
after brain injury", JOURNAL OF
NEUROINFLAMMATION, BIOMED CENTRAL
LTD., LONDON, GB, vol. 10, no. 1, 17 December
2013 (2013-12-17), page 155, XP021171420, ISSN:
1742-2094, DOI: 10.1186/1742-2094-10-155**
• **CAROLYN C. ALLRED ET AL: "A novel ELISA for
measuring CD36 protein in human adipose
tissue", JOURNAL OF LIPID RESEARCH, vol. 52,
no. 2, 1 February 2011 (2011-02-01), pages
408-415, XP055475929, US ISSN: 0022-2275, DOI:
10.1194/jlr.M008995**
• **Elisa Basics: "ELISA ELISA Basics Guide", , 31
December 2017 (2017-12-31), XP055475932,
Retrieved from the Internet:
URL:https://www.bio-rad-antibodies.com/sta
tic/2017/an-introduction-to-elisa/elisa-ba
sics-guide.pdf [retrieved on 2018-05-16]**

## Description

### Field of the invention

The present invention relates to a diagnostic method for the Nox2 protein determination in a biological sample and related kit.

### Technological background

There are numerous studies that identify the enzyme NADPH oxidase as one of the main cellular enzymes capable of generating reactive oxygen species (ROS). At the moment, 7 different isoforms belonging to the NADPH oxidase family are known, such as Nox1, Nox2, Nox3, Nox4, Nox5 and DUOX 1 and 2, characterized by various structural and functional analogies. These catalytic units may be present simultaneously in the same cells or in different cells within the same tissue where they probably perform complementary or partially overlapping functions according to the different physiological states in which they are found (Bedard K. and Krause K, Physiol. Rev. (2007) 87: 245-313).

The most studied Nox isoform is Nox2, also known as phagocyte oxidase, mainly found in phagocytic cells of the immune system, such as neutrophils and macrophages. In these cells, Nox2 produces superoxide anion and, secondarily, hydrogen peroxide within the phagosomes, allowing these cells to phagocytize and kill bacterial cells during infections.

In recent years, the role of ROS in blood circulation and their interaction with vessel tone modulation systems has received much interest from researchers working on the phenomenon of atherosclerosis (Brodsky S.V. et al., Am. J. Physiol. (2004) 286: H1910-1915; Dworakowsky R. et al., Pharmacol. Reports (2008) 60:21-28; Liming Jin and Burnett A.L., Asian J. Androl. (2008) 10:6-13; Kitas G.D. and Erb N., Rheumatology (2003) 42:607-613; Martino F. et al., Pediatrics (2008) 122:e648-e655).

The presence of the enzymes of the Nox family can be detected in cultured or isolated cells or in tissues through the analysis of their enzymatic activity, i.e. mainly through the indirect analysis of ROS production.

The direct analysis of these molecular species is particularly difficult to perform because the great majority of them are extremely reactive and their life as radicals is very short. The only method that allows the direct and specific determination of free radicals is spin resonance, but its in vivo use is limited by two main factors. First, the lack of spin trap substances approved for use in humans. Moreover, its sensitivity, however high, allows to detect only the levels of less reactive radicals that remain longer in the sample.

The reaction of ROS with cellular components and biological fluids causes the formation of DNA derivatives, lipid peroxides, amino acid modifications that could be considered biological markers if there was a precise correlation between their level and a specific pathology, which at the moment has not yet been verified. Despite this, some biological markers are considered more reliable than others by those skilled in the art (Halliwell B. and Whiteman M., Br. J. Pharmacol. (2004) 142:231-255). Below are the most used by those skilled in the art.

The analysis of DNA and protein peroxidation products is one of these methods, but it still presents considerable methodological problems (Halliwell B. and Whiteman M., *supra*).

Another method used is the volatile hydrocarbon determination (ethane, pentane, and ethylene) in the *in vivo* expired air (Dale et al., Free Radic. Res. (2003) 37:815-821, US Patent 6,254,547).

Then, there are methods that are used for the analysis of peroxidation levels in cells, for example in leukocytes, or for research purposes exclusively in cell cultures, and which are based on the use of fluorescent probes such as dichlorofluorescein acetate (DCFH), dihydrorodamine (DHR), dihydroetidine (DHE), luminol and lucigenin, which however have considerable problems of sensitivity and stability.

Finally, there are methods for the *in vitro* study of NADPH oxidase activity, such as the inhibition of cytochrome c reduction by the enzyme superoxide dismutase (SOD) in the presence of $O^{2-}$ for the measurement of NADPH oxidase activity in "cell free" systems and cell culture supernatant (Cross A.R., Ericson R.W., and Curnute J.T., Biochem. J. (1999) 341:251-255; Teufelhofer O. et al., Toxicol. Sci. (2003) 76:376-383).

The expression of NADPH oxidase can also be measured by analyzing the presence in the cells or tissues of their proteins, for example by expressing the respective genes with the polymerase chain reaction (PCR) technique.

Other indirect methods are based on the measurement of *in vivo* lipid peroxidation. Lipid peroxidation is a rather complex process that generates a number of compounds in variable quantities and whose determination is generally considered as an oxidative stress index.

Although many studies conducted to date have shown a parallelism between elevated isoprostane levels in plasma or urine and hyperglycaemia and hypercholesterolemia in humans, the EIA and ELISA assays may not correctly estimate the true prostaglandin F levels present in the sample due to cross-reactivity of the antibodies used to the central nucleus of the different prostaglandins F (64 different molecular species) potentially present in the tested sample.

The present inventors have previously developed a method for detecting the soluble Nox2 peptide (sNox2-dp) in a biological sample as described in US patent application US 2012/0156704. Although the method is reliable, this did not allow to distinguish the pathological sNox2-dp values compared to physiological values. Other assays for measuring the levels of Nox2 protein are disclosed i.a. from Paclet et al, Biochemical Journal 2004, 382(3):981-986; Campion et al, Biochemie 2007, 89(9):1145-1158; Cooney et al, Journal of Neuroinflammation 2013, 10(1):155. Allred et al, Journal of Lipid Research 2011, 52(2):408-415 discloses the use of normalized samples in immunoassays.

**Summary of the invention**

The object of the present invention is to provide an improved *in vitro* detection method of the Nox2 protein compared to the state of the art, which allows to effectively discriminate healthy subjects and sick subjects suffering from pathologies characterized by high oxidative stress, i.e. a high amount of Nox2.

According to the invention, this object is achieved by the method specified in the following claims, which are intended as an integral part of the present description.

In one embodiment, the present disclosure relates to a diagnostic method for quantitative detection of Nox2 protein, preferably the soluble portion of Nox2 protein (sNox2-dp), in a biological sample comprising the following operations:

i) normalizing the protein content of the biological sample to a value of 1 μg of protein per 100 μl of sample;
ii) contacting the normalized biological sample with a monoclonal anti-Nox2 detection antibody, in which the detection antibody is directly conjugated with a detection marker, and
iii) detecting the formation of the Nox2 protein-detection antibody complex.

In one embodiment, the present invention relates to a kit for implementing the diagnostic method for quantitative detection of Nox2 protein, preferably the soluble portion of Nox2 protein (sNox2-dp), in a biological sample, in which the kit includes:

i) a solid phase suitable for conducting a diagnostic test,
ii) a monoclonal anti-Nox2 detection antibody directly conjugated to a detection marker, wherein the detection antibody recognizes an epitope of the Nox2 protein present in the amino acid portion of Nox2 having the sequence as set forth in SEQ ID No.:1,
iii) a set of calibrators containing known scalar concentrations of the amino acid portion of NOx2 protein as set forth in SEQ ID No.:1, or an amino acid sequence having at least 90%, preferably at least 95%, more preferably at least 98%, of identity with SEQ ID No.:1, wherein the known scalar concentrations are between 0 and 400 pg/ml,
iv) instructions for use and,
v) a buffer solution for diluting the biological sample.

**Brief description of the drawings**

The invention will now be described, by way of example only, with reference to the figures of the attached drawings, in which:

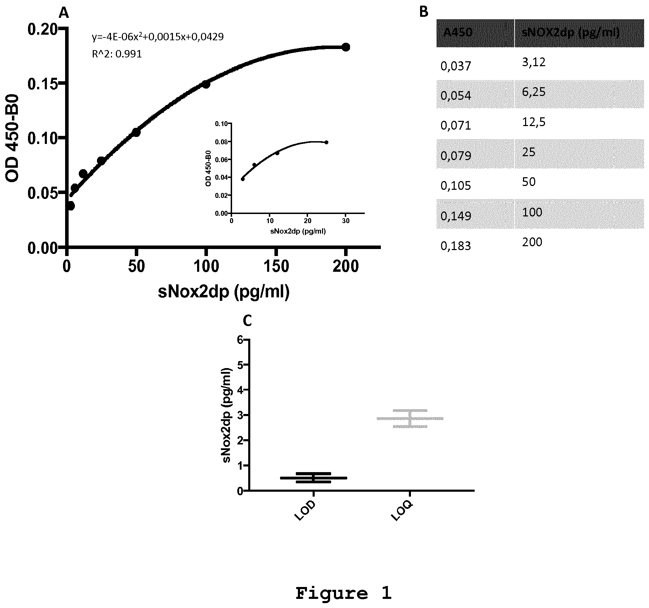

- **Figure 1: Interpolation of the standard curve and concentration limits.** The standard curve was obtained with sNOX2dp peptide concentrations in the range 3.12-200 pg/ml. The curve is a polynomial second-degree function represented by the equation

$$y=-4E-06x^2+0.0015x+0.0429$$

where y is the absorbance measured at 450 nm and x is the sNox2dp concentration expressed in pg/ml (A). Table of averages of absorbance values (OD450-B0) and related concentrations (B). LOD and LOQ values expressed as mean ± SD (n=3).

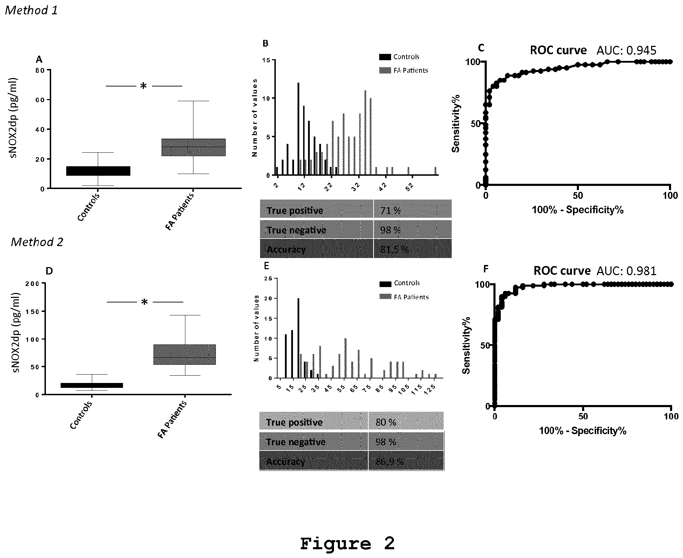

- **Figure 2: Levels of sNox2dp measured with Method 1 and Method 2.** Application of Method 1 and Method 2 in patients with atrial fibrillation AF (n=80) and control subjects (n=50) (Figure 2A and D). Distribution of concentrations for the two populations and percentages of sensitivity, specificity and accuracy (Figure 2B and E) and ROC analysis (Figure 2C and F).

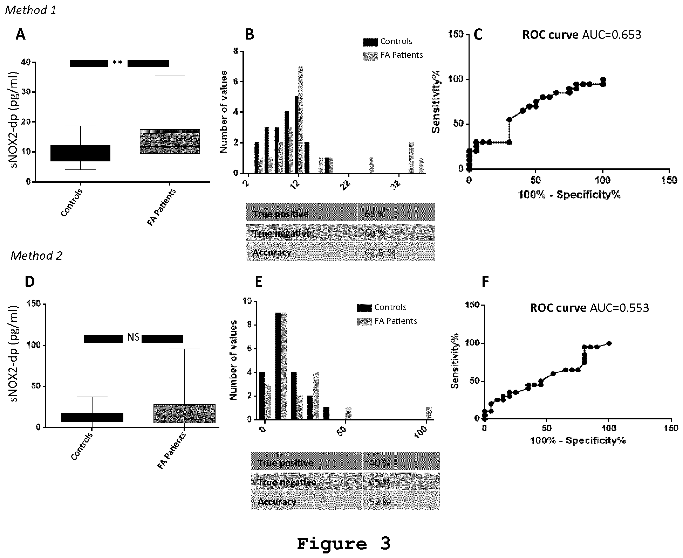

- **Figure 3: Comparison of sNox2dp levels measured with Method 1 and Method 2 with or without sample normalization.** Measurement of sNox2dp levels in a subset of AF patients (n=20) and healthy subjects (n=20) in a normalized sample for protein content (1 μg/100 μl) and in the presence of the anti-Nox2 antibody and HRP conjugated secondary antibody, (Figure 3A) and in a non-normalized sample for protein content (100 μl) and in the presence of the HRP conjugated anti-Nox2 antibody (Figure 3D). Distribution of concentrations for the two populations and percentages of sensitivity, specificity and accuracy (Figure 3B and E) and ROC analysis (Figure 3C and F).

**Detailed description of the invention**

In the following description, a number of specific details are provided to allow a full understanding of the embodiments. The embodiments can be practiced without one or more of these details. In other cases, structures, materials or operations are not shown or described in detail to avoid obscuring the aspects of the embodiments.

The reference in the detailed part to "an embodiment" or "the embodiment" means that a particular feature, structure, or characteristic described in connection with the embodiment is included in at least one of the embodiments. Thus, when the phrases "in one embodiment" or "in the embodiment" appear at various points in this specific part, these are not necessarily all referring to the same embodiment. Furthermore, a particular feature, structure, or characteristic can be combined in any manner in one or more embodiments.

The references provided here are the only ones for convenience and do not interpret the purpose or meaning of the embodiments.

The present invention relates to a diagnostic method for the quantitative detection of Nox2 protein, preferably the soluble portion of Nox2 protein (sNox2-dp), in a biological sample comprising the following operations:

i) normalizing the protein content of the biological sample to a value of 1 μg of protein per 100 μl of sample;
ii) contacting the normalized biological sample with a monoclonal anti-Nox2 detection antibody, in which the detection antibody is directly conjugated with a detection marker, and
iii) detecting the formation of the Nox2 protein-detection antibody complex.

This method, in view of the characteristics of normalization of the protein content of the biological sample to be analyzed and of use of an anti-Nox2 detection antibody directly conjugated to a detection marker, allows to carry out a very accurate determination of the Nox2 protein contained in the biological sample, such as to allow a correct discrimination between healthy subjects and sick subjects.

It is in fact known in the art that subjects affected by a state of high oxidative stress, which involves a high amount of Nox2 (specifically its soluble portion) in the blood, present a greater risk of appearance or worsening of metabolic pathologies, such as diabetes, hypercholesterolemia, hyperlipidemia, cardiovascular system diseases such as arterial hypertension, atherosclerosis, cardiac hypertrophy, myocardial infarction, atrial fibrillation, and/or inflammatory diseases, such as rheumatoid arthritis, or sepsis. The accurate determination of the oxidative stress level in these subjects is particularly useful for monitoring the respective therapies (antiinflammatory drugs, hypoglycemic agents, or drugs useful for the treatment of cardiovascular diseases such as statins, ACE-inhibitors or beta-blockers) in order to verify their effectiveness and/or correct the therapies themselves by increasing or decreasing the quantity of administered drugs.

The method developed by the present inventors overcomes the disadvantages of the methods known in the art for the determination of oxidative stress as - in view of the normalization of the protein content of the biological sample to be analyzed at a value of 1 μg of protein per 100 μl of sample - allows to reduce the risks of overestimation and/or underestimation of the quantity of detected Nox2. The normalization of the protein content of the biological sample also allows to reduce the interferences in the detection due to an excessive quantity of analyte/protein to be analyzed. In fact, by normalizing the sample for protein content and subsequently choosing a low protein concentration (i.e. 1 μg/100 μl) the quantification of Nox2 contained in the biological sample is substantially correct, allowing discrimination between healthy subjects and sick subjects suffering from diseases characterized by oxidative stress.

The diagnostic method described here also provides for direct conjugation of the anti-Nox2 detection antibody with a detection marker. In this way, by avoiding the operation of addition of a secondary antibody capable of recognizing the monoclonal anti-Nox2 antibody bound to the Nox2 protein contained in the biological sample, the diagnostic test time as well as the potential problems of cross-reactivity of the secondary antibody with the antigenic components of the sample are reduced. This is an advantage considering the application of the method in diagnostic field in the population screening programs where it is necessary to optimize the time, providing however reliable results.

In a preferred embodiment of the present diagnostic method, the anti-Nox2 detection antibody directly conjugated to a detection marker was generated against a Nox2 epitope present in the Nox2 portion having amino acid sequence as sets forth in SEQ ID No.: 1. In a further preferred embodiment, the monoclonal anti-Nox2 detection antibody is generated by the hybridoma PP7H10 deposited at the CBA-ICLC of Genoa with deposit number PD17002.

In one or more embodiments, the method object of the present description further comprises the following operations:

iii) constructing a calibration curve by using a calibrator set containing known scalar concentrations of the amino acid portion of Nox2 protein as sets forth in SEQ ID No.:1, or an amino acid sequence having at least 90%, preferably at least 95%, more preferably at least 98%, of identity with SEQ ID No.:1, and

iv) quantitatively determining the Nox2 protein in the biological sample by reporting the result of the operation ii) on the calibration curve obtained in the operation iii).

The realization of this calibration curve allows to determine, in an accurate way, the amount of Nox2 protein contained in the biological sample through the use of the anti-Nox2 detection antibody. Preferably, the construction of the calibration curve involves the use of a blank and at least 4, preferably at least 7, calibrators. These calibrators have a concentration of the amino acid portion of Nox2 protein as sets forth in SEQ ID No.:1 (or an amino acid sequence having at least 90%, preferably at least 95%, more preferably at least 98%, of identity with SEQ ID No.: 1) between 0 and 400 pg/ml, preferably between 0 and 200 pg/ml. By way of example, the calibrators may comprise the following concentrations of the amino acid portion of Nox2 protein as sets forth in SEQ ID No.:1: 0, 12.5, 25, 50, 100, 200, 300, 400 pg/ml; or 0, 3.12, 6.25, 12.5, 25, 50, 100, 200 pg/ml.

In one or more embodiments, the detection marker directly conjugated to the anti-Nox2 detection antibody is selected among radioisotopic, enzymatic, fluorimetric, bioluminescent, chemiluminescent tracers. Such tracers as well as the related antibody conjugation techniques usable in diagnostic assays are widely known in the art and do not require further details herein. Among the detection markers, we can cite as an example: fluorescein (FITC), rodamine, phycoerythrin (PE), tetramethylrodamine and green fluorescent protein (GFP) that are used as tracers in immunofluorescence assays; horseradish peroxidase enzyme (HRP), alkaline phosphatase, glucose oxidase, beta-galactosidase and luciferase (Hempen C., Anal. Bioanal. Chem. (2006) 384:572-583). Depending on the detection marker, those skilled in the art will use the common general knowledge of the field to develop the most suitable detection method.

In one or more embodiments, the diagnostic method described herein is a direct immunoassay. Particularly, on a solid phase (consists of polypropylene, for example), the sample to be analyzed containing the soluble fraction of Nox2 (appropriately normalized for protein content) or a calibrator is added, obtaining an adsorption of the Nox2 contained in the biological sample or the calibrator directly on the solid phase.

In a particularly preferred embodiment, the method involves the use of the direct ELISA technique, which represents, to date, the most used analytical technique in laboratory medicine, which includes a vast repertoire of analytes that are analyzed by an increasingly widespread range of devices. Direct ELISA is a very sensitive method and allows to save reagents and time compared to other assay modes. Furthermore, this method has the advantage of being automated, leading to further improvements in the reliability, accuracy, sensitivity and cost of the reagents, allowing the use of the same in population screening programs.

In one or more embodiments, the biological sample to be analyzed is selected from serum, plasma, cell supernatants.

According to an embodiment of the present invention, the method described herein is implemented using a kit comprising:

i) a solid phase suitable for conducting a diagnostic assay, preferably a direct immunodiagnostic assay,

ii) a monoclonal anti-Nox2 detection antibody directly conjugated to a detection marker, wherein the detection antibody recognizes an epitope of the Nox2 protein present in the amino acid portion of Nox2 having the sequence as set forth in SEQ ID No.:1,

iii) a set of calibrators containing known scalar concentrations of the amino acid portion of Nox2 protein as sets forth in SEQ ID No.:1, or an amino acid sequence having at least 90%, preferably at least 95%, more preferably at least 98%, of identity with SEQ ID No.:1, wherein the known scalar concentrations are between 0 and 400 pg/ml,

iv) instructions for use providing for normalising the protein content of the biological sample to a value of 1 μg of protein per 100 μl of sample before the determination of the Nox2 protein and,

v) a buffer solution for diluting the biological sample.

In one embodiment, the detection antibody is generated from the hybridoma PP7H10 deposited at the CBA-ICLC of Genoa with deposit number PD17002.

In one or more embodiments, the set of calibrators contained in the kit comprises a blank and at least 4, preferably at least 7, calibrators, having a concentration of the amino acid portion of Nox2 protein as sets forth in SEQ ID No.:1 (or an amino acid sequence having at least 90%, preferably at least 95%, more preferably at least 98%, of identity with SEQ ID No.:1) ranging from 0 to 200 pg/ml.

The instructions for use provide information useful for conducting the diagnostic method and particularly indicate that the biological sample must be diluted with the buffer solution before the assay is conducted in order to normalize the protein content of the sample to a value of 1 μg of protein per 100 μl of sample.

The instructions for use also provide the information necessary for the realization of the calibration curve and its interpolation. Preferably, the calibration curve is a polynomial second-degree function represented by the equation:

$$y=-4E-06x^2+0.0015x+0.0429 \qquad (eq.\ 1)$$

where y is the absorbance measured at 450 nm and x is the concentration of sNox2dp (SEQ ID No.:1) expressed in pg/ml.

### Materials and Methods

The human Nox2 peptide with amino acid sequence AERIVRGQTAESLAVHNITVCEQKISEWGKIKECPIPQFAGNPPM (amino acids 224-268 of the human sequence www.ncbi.nlm.nih.gov/protein/NP_000388.2 - SEQ ID No.:1) having a molecular weight of 4.9 kDa, and the anti-Nox2 antibody generated against this peptide (hybridoma PP7H10) were produced by the Biotechnology Center (CRIBI) of the University of Padua. The hybridoma PP7H10 of the anti-Nox2 antibody was deposited on 17 October 2017 at the CBA-ICLC deposit center in Genoa with deposit number PD17002.

All reagents necessary for the development of the Nox2 determination method are present in the ELISA Starter Accessory Kit (EIA code 101, Bethyl Laboratories). Sulfuric acid (H2SO4) was purchased from Merck (code 30148297). To verify the specificity of the examined peptide and the Nox2 antibody (XYZ hybridoma), the "Basic Local Alignment Search Tool (BLAST)" program was used to find similar regions between the amino acid sequence of the Nox2 taken into consideration and the other Nox isoforms (Nox1, Nox3, Nox4 and Nox5), xanthine oxidase and myeloperoxidase. The similarity percentage of the peptide and the possible combinations of 6 identical amino acids for antibody recognition are shown in Table 1.

**Table 1**

| Enzyme | Nox2 | |
|---|---|---|
| | * Identified peptide sequence | ** identification of 6 aa aligned necessary for antibody binding |
| Nox1 | 14/45 (31%) | 0/154 (0%) |
| Nox3 | 23/45 (51%) | 2/154 (1.3%) |
| Nox4 | 4/45 (8.8%) | 0/154 (0%) |
| Nox5 | No significant similarity was found | No significant similarity was found |
| Xanthine oxidase | No significant similarity was found | No significant similarity was found |
| Myeloperoxidase | 4/45 (8.8%) | 0/154 (0%) |
| * identity: it is the number of amino acids identical for the alignment length of the 45 amino acids of the identified Nox2 peptide; the identity percentage resulting from the 45 amino acids of the identified Nox2 peptide is shown in brackets ** the number of possible combinations of 6 aligned amino acids that can be recognized by the antibody. | | |

The results reported in Table 1 show low identity percentages between the amino acid sequence consists of 45 aa, recognized by the anti-Nox2 antibody, and the respective amino acid sequences of the other Nox isoforms. The exception is Nox3 which has a 51% identity. However, the analysis of the number of possible combinations of 6 aligned amino acids, necessary for binding with the antibody, shows a greatly reduced recognition percentage. These data allow us to conclude that the antibody we have produced recognizes the Nox2 in a highly specific way.

### Intra-assay and inter-assay repeatability

The repeatability of the analytical method was ascertained by measuring the intra-assay and inter-assay percentage variation coefficient (%VC).

For the evaluation of intra-assay variability, the average of absorbance values at 450 nm (n=10 repetitions for each point of the standard curve in the range 0-200 pg/ml), the standard deviation (SD) and the %VC were calculated. The intra-assay evaluation was repeated for 3 days (Table 2).

**Table 2**

| Intra-assay accuracy | | | | | | | | | |
|---|---|---|---|---|---|---|---|---|---|
| Concentration (pg/ml) | | 0 | 3.12 | 6.25 | 12.5 | 25 | 50 | 100 | 200 |
| Day 1 | Average of absorbance values (450 nm) 10 standard curves | 0.097 | 0.134 | 0.150 | 0.159 | 0.177 | 0.185 | 0.217 | 0.223 |
| | SD | 0.005 | 0.017 | 0.014 | 0.028 | 0.016 | 0.021 | 0.012 | 0.020 |
| | %VC | 5.15 | 12.68 | 9.33 | 17.61 | 9.03 | 11.35 | 5.52 | 8.58 |
| Day 2 | Average of absorbance values (450 nm) 10 standard curves | 0.095 | 0.134 | 0.154 | 0.172 | 0.175 | 0.192 | 0.219 | 0.233 |
| | SD | 0.005 | 0.011 | 0.028 | 0.019 | 0.013 | 0.020 | 0.006 | 0.024 |
| | %VC | 5.26 | 8.20 | 18.18 | 11.04 | 7.42 | 10.41 | 2.73 | 10.30 |
| Day 3 | Average of absorbance values (450 nm) 10 standard curves | 0.096 | 0.133 | 0.148 | 0.157 | 0.174 | 0.190 | 0.217 | 0.227 |
| | SD | 0.005 | 0.017 | 0.020 | 0.010 | 0.016 | 0.010 | 0.006 | 0.016 |
| | %VC | 5.21 | 12.78 | 13.51 | 6.36 | 9.19 | 5.26 | 2.76 | 7.04 |
| Overall intra-assay accuracy | | | | | | | | | |
| | | 5.21 | 11.22 | 13.67 | 11.67 | 8.54 | 9.00 | 6.04 | 9.59 |
| Overall intra-assay accuracy (working range 0-200 pg/ml) | | | | | | | | | |
| | | 8.95 | | | | | | | |

For the assessment of inter-assay variability, the average of absorbance values at 450 nm over 3 days, SD and %VC were calculated (Table 3). The %VC values in the intra- and inter-assay studies are less than 10%.

**Table 3**

| Inter-assay accuracy | | | | | | | | |
|---|---|---|---|---|---|---|---|---|
| Concentration (pg/ml) | 0 | 3.12 | 6.25 | 12.5 | 25 | 50 | 100 | 200 |
| Average of absorbance values (450 nm) 3 days | 0.096 | 0.133 | 0.150 | 0.162 | 0.175 | 0.189 | 0.217 | 0.227 |
| SD | 0.005 | 0.016 | 0.020 | 0.019 | 0.015 | 0.017 | 0.008 | 0.020 |
| %VC | 5.20 | 12.00 | 13.30 | 11.7 | 8.57 | 8.9 | 3.68 | 8.8 |
| Overall inter-assay accuracy (working range 0-200 pg/ml) | | | | | | | | |
| 9.01 | | | | | | | | |

*Standard curve interpolation*

The standard curve was obtained by preparing concentration of sNox2dp peptide as sets forth in SEQ ID No.:1 in the range of 3.12 - 200 pg/ml after absorbance subtraction at 0 pg/ml (B0). The curve is a polynomial second-degree function represented by the equation:

$$y = -4E-06x^2 + 0.0015x + 0.0429 \qquad (eq.\ 1)$$

where y is the absorbance measured at 450 nm and x is the concentration of sNox2dp expressed in pg/ml (Figure 1A).

The correlation coefficient is 0.991.

The averages of absorbance values (OD450-B0) and related concentrations are shown in Figure 1B.

*Determination of detection limit and quantification limit.*

The detection limit (LOD) and the quantification limit (LOQ) are an important feature of the analytical method that identifies the lower limit of concentration below which Nox2 cannot be detected (LOD) or quantified (LOQ) with sufficient statistical probability.

LOD and LOQ were calculated according to the EURACHEM working group method as published (Weber D, Free Radical Research (2012) 46:276-285) by calculating the blank mean ($x_B$) and adding multiples of the blank standard deviation ($s_B$):

$$LOD = x_B + 3\ s_B \quad (eq.\ 2)$$

$$LOQ = x_B + 9\ s_B \quad (eq.\ 3)$$

For the present method, LOD is $0.51 \pm 0.15$ pg/ml while LOQ is $2.87 \pm 0.31$ pg/ml (Figure 1C).

*Application of the method on patients with atrial fibrillation*

*Serum collection*

The blood was collected in Vacutainer tubes without anticoagulant (5 ml, red cap) which were subsequently centrifuged at 300g for 10 minutes. The supernatant was aspirated and stored at -80°C until use.

*Patient recruitment*

Recruited patients who were included in the study were 80 patients with Atrial Fibrillation (AF) of the Oral Anticoagulant Therapy Center (TAO) of the Department of Internal Medicine and Medical Specialities of the Sapienza University of Rome. 50 healthy subjects, paired for age and sex, were recruited as a control group. The clinical features of patients and controls are shown in Table 4.

**Table 4**

|  | Controls | Patients | p* |
|---|---|---|---|
| Age (years) | $70.0 \pm 11.4$ | $77.2 \pm 6.0$ | 0.296 |
| Persistent/permanent AF (%) | 0 | 69.0 | <0.001 |
| Women (%) | 33.3 | 45.2 | 0.340 |
| Hypertension (%) | 0 | 92.9 | <0.001 |
| Diabetes mellitus (%) | 0 | 14.3 | <0.001 |
| IM history/cardiac revascularization (%) | 0 | 26.2 | <0.001 |
| Heart failure (%) | 0 | 9.5 | <0.001 |
| History of stroke/TIA (%) | 0 | 23.8 | <0.001 |
| Statins (%) | 0 | 54.8 | <0.001 |

All the subjects included in the study signed an informed consent; they were subjected to the comparative analysis of serum Nox2 levels detected both through the method previously described in US 2012/0156704 (hereinafter **Method 1**) and the present method (hereinafter **Method 2**).

*Laboratory analysis*

For the analysis of Nox2 with Method 2, the protein content of each serum sample was evaluated and the peptide concentration in the sample with final protein concentration of 1 μg/100 μl was evaluated. For the analysis of Nox2 with method 1 100 μl of serum were directly analyzed.

*Determination of Nox2 in human serum by Method 2*

The first stage of the ELISA method involves 1) the binding of Nox2 peptide at known scalar concentrations and 2) the binding of Nox2 contained in the sample to be analyzed to a 96-well polypropylene ELISA plate. Specifically, the serum sample containing the antigen was plated in the presence of the carbonate/bicarbonate buffer, which doesn't contains other proteins that could compete with the target antigen for solid phase absorption.

For the quantitative determination of Nox2, a standard curve is obtained by using the following scalar concentrations of Nox2 peptide (SEQ ID No.: 1) prepared by diluting these quantities in a buffer solution (Carbonate/bicarbonate buffer capsules - Coating Buffer; Sigma Chemical #C3041; EIA 101; Bethyl Laboratories): 0, 3.12, 6.25, 12.5, 25, 50, 100, 200 pg/ml.

In serum samples, the protein concentration is determined by the Bradford method (Bradford MM. Analytical Biochemistry (1976), 72:248-254) widely known in the art and which does not need further details here.

Subsequently, each sample is diluted in Coating Buffer (at a concentration of 0.05M and pH 9.6) until it reaches a final protein concentration of 1 $\mu$g in 100 $\mu$l.

At the end of the preparation, 100 $\mu$l of standard or sample are added to each well and the plate is incubated overnight at 4°C. At the end of the incubation, the content is aspirated and the wells are washed three times with a washing solution (50 mM Tris buffered saline, pH 8.0, 0.05% Tween20, Sigma Chemical # T9039; EIA 101 Bethyl Laboratories).

Thereafter, 200 $\mu$l of blocking solution, Blocking Buffer (50 mM Tris buffered saline, pH 8.0, 1% BSA, Sigma Chemical # T6789; EIA 101; Bethyl Laboratories) are added to each well for 120 minutes at room temperature. At the end of the incubation, 3 washings are performed for each well of the plate with the washing solution (50 mM Tris buffered saline, pH 8.0, 0.05% Tween20, Sigma Chemical #T9039; EIA 101 Bethyl Laboratories).

Once the washings are complete, the HRP conjugated anti-sNox2dp antibody is diluted (1:5000 in Sample/Conjugate Diluent, 0.5 ml 10% Tween 20 to 100 ml ELISA Blocking Buffer) and 100 $\mu$l of antibody solution are dispensed into each well for 120 minutes at room temperature. After incubation, the contents of each well are aspirated and the wells are washed 5 times with washing solution (50 mM Tris buffered saline, pH 8.0, 0.05% Tween20, Sigma Chemical # T9039; EIA 101 Bethyl Laboratories), and 100 $\mu$l of substrate are added (TMB One Component HRP Microwell Substrate, EIA 101 Bethyl Laboratories) for 30 minutes at 37°C. At the end of the incubation, 100 $\mu$l of 2M $H_2SO_4$ (Merck code 30148297) are added with a 1:4 dilution.

The reading of the obtained staining was spectrophotometrically carried out at 450 nm. The results are extrapolated from the standard curve obtained with the scalar peptide concentrations.

*Statistic analysis*

Data are reported as mean $\pm$ standard deviation (SD). The accuracy (variation coefficient, VC) was calculated from the ratio between SD and average and expressed as a percentage. The comparison between the variables was made using the Student's t test, accepting the value p <0.05 as significant.

ROC ("receiver operating characteristic") curves are graphically reproduced and used to evaluate the optimal threshold values for the different sensitivity and specificity values. The area under the curve (AUC) values are evaluated as follows: non-informative AUC = 0.5; inaccurate 0.5 < AUC $\leq$ 0.7; moderately accurate 0.7 < AUC $\leq$ 0.9; highly accurate 0.9 < AUC $\leq$ 1; perfect AUC = 1.

Sensitivity was calculated as the proportion of patients with a positive test among all patients with atrial fibrillation, i.e. the proportion of true positives; the specificity was calculated as the proportion of subjects with negative test among all the control subjects, i.e. the proportion of true negatives; accuracy was calculated as the proportion of correctly classified patients.

*__Results__*

The application of Method 1 shows a high concentration of the peptide in patients with AF compared to control subjects (27.7$\pm$8.7 pg/ml vs. 12.0$\pm$4.7 pg/ml, p<0.001) (Figure 2A). Similarly, the application of Method 2 shows a high concentration of the peptide in patients with AF compared to control subjects (72.9$\pm$26.3 pg/ml vs. 16.8$\pm$5.7, p<0.001) (Figure 2D).

By evaluating the distribution of the concentrations for the two populations it should be noted that the histograms for both the two ELISA methods are superimposed but the overlap area is substantially narrower for Method 2 compared to Method 1 (Figure 2B and E) thus allowing the discrimination between the two populations in a more precise way. This turns into a higher percentage of true positives (80% in Method 2 vs. 71% in Method 1) and greater accuracy (86.9% in Method 2 vs. 81.5% in Method 1). Finally, the results of the ROC analysis confirm that Method 2 (AUC 0.981) is more accurate than Method 1 (AUC 0.945) (Figure 2D and F).

Moreover, comparative experimental tests show that the application of a variant of Method 1, in which a normalized sample for protein content and the anti-Nox2 antibody not directly conjugated with the detection marker are used, shows a difference in concentration of sNOX2dp in patients with AF compared to control subjects (15.11±9.5 pg/ml vs. 9.8±3.6 p=0.02) (Figure 3A). However, by evaluating the distribution of the concentrations for the two populations it should be noted that the histograms are almost completely overlapping (Figure 3B), thus not allowing the discrimination between the two populations in a precise manner. This results in a lower accuracy of the method (62.5%) also confirmed by the results of the ROC analysis which show that the application of the variant of Method 1, that is the use of a normalized sample (AUC 0.653), is an inaccurate method (Figure 3C).

The application of a variant of Method 2, i.e. using a non-normalized sample for protein content and the anti-Nox2 antibody directly conjugated to the detection marker, does not show significant differences between the peptide concentration in patients with AF compared to the control subjects (19.08±21.5 pg/ml vs. 12.79±9.8 pg/ml, p=0.242) (Figure 3D). By evaluating the distribution of the concentrations for the two populations it is evident that the histograms are completely overlapping (Figure 3E) and that the method is poorly accurate (52%). Finally, the results of the ROC analysis confirm that the application of the variant of Method 2 in a non-normalized sample (AUC 0.553) is inaccurate (Figure 3F).

# EP 3 495 821 B1

Trattato di Budapest sul Riconoscimento Internazionale del Deposito di Microorganismi per motivi di Brevetto

Formulario Internazionale

| | |
|---|---|
| Spett. le<br>Università degli Studi di Roma<br>"La Sapienza"<br>Piazzale Aldo Moro 5<br>00185 Roma<br>Italy | Ricevuta di deposito originale<br>emessa ai sensi della Regola 7.1<br>dalla Autorità Internazionale di Deposito<br>sotto specificata |

**I. Identificazione del microrganismo (ibridoma o linea cellulare)**

| Riferimento identificativo fornito dal<br>Depositante:<br>**PP7H10** | Numero di accesso assegnato dall'Autorità<br>Internazionale di Deposito: **PD 17002** |
|---|---|

**II. Descrizione scientifica e/o designazione tassonomica proposta**

La linea cellulare/ibridoma identificata al punto I era accompagnato da:

☑ una descrizione scientifica    ☐ una designazione tassonomica proposta

**III. Ricevuta e accettazione**

Questa Autorità Internazionale di Deposito accetta l'ibridoma identificato al punto I, ricevuto il giorno **17 Ottobre 2017** (data del deposito originale)

**IV. Ricevuta di richiesta di conversione**

La linea cellulare identificata al punto I è stata ricevuta da questa Autorità Internazionale di Deposito il giorno                    (data del deposito originale)

una richiesta di convertire il deposito originale in deposito ai sensi del trattato di Budapest é stata ricevuta il giorno                    (data della richiesta di conversione)

**V. Autorità Internazionale di Deposito**

| Nome:<br>Centro di Biotecnologie Avanzate (CBA)<br>Interlab Cell Line Collection<br><br>Indirizzo:<br>L.go R. Benzi, 10<br>16132 GENOVA  ITALIA | Firma del Rappresentante Legale<br>dell'Autorità Internazionale di Deposito o di<br>un ufficiale autorizzato:<br><br>Dr. Carlo Castelli<br>Il liquidatore CBA |

Formulario BP/4 (pagina singola)

## Claims

1.  A diagnostic method for the quantitative detection of Nox2 protein in a biological sample, comprising the following operations:

    i) normalizing the protein content of the biological sample to a value of 1 $\mu$g of protein per 100 $\mu$l of sample,
    ii) contacting the normalized biological sample with a monoclonal anti-Nox2 detection antibody, in which the detection antibody is directly conjugated with a detection marker, and
    iii) detecting the formation of the Nox2 protein-detection antibody complex.

2.  The method according to claim 1, wherein the detection antibody recognizes an epitope of the Nox2 protein present in the amino acid portion of Nox2 having the sequence as sets forth in SEQ ID No.:1.

3.  The method according to claim 1 or claim 2, wherein the operation i) of normalization of the protein content of the biological sample is carried out by diluting the biological sample with a buffer solution.

4.  The method according to any one of the previous claims, in which the detection marker is selected among radioisotopic, enzymatic, fluorimetric, bioluminescent, chemiluminescent tracers.

5.  The method according to any one of the previous claims, wherein the method further comprises the following operations:

    iv) constructing a calibration curve by using a set of calibrators containing known scalar concentrations of the amino acid portion of Nox2 protein as sets forth in SEQ ID No.:1, or an amino acid sequence having at least 90%, preferably at least 95%, more preferably at least 98%, of identity with SEQ ID No.:1, and
    v) determining the quantity of the Nox2 protein in the biological sample by reporting the result of the operation iii) on the calibration curve obtained in the operation iv).

6.  The method according to claim 5, wherein the known scalar concentrations of the amino acid portion of Nox2 protein as sets forth in SEQ ID No.:1, or an amino acid sequence having at least 90%, preferably at least 95%, more preferably at least 98%, of identity with SEQ ID No.:1, contained in the calibrators are between 0 and 400 pg/ml, preferably between 0 and 200 pg/ml.

7.  The method according to claim 5 or claim 6, in which the calibrator set comprises a blank and at least 4, preferably at least 7, calibrators.

8. The method according to any one of the previous claims, in which the detection antibody is generated by the hybridoma PP7H10 deposited at the CBA-ICLC of Genoa with deposit number PD17002.

9. The method according to any one of the previous claims, in which the biological sample is selected from serum, plasma, cellular supernatant.

10. A kit for the quantitative determination of the Nox2 protein in a biological sample, comprising:

   i) a solid phase suitable for conducting a diagnostic test,
   ii) a monoclonal anti-Nox2 detection antibody directly conjugated to a detection marker, wherein the detection antibody recognizes an epitope of the Nox2 protein present in the amino acid portion of Nox2 having the sequence as set forth in SEQ ID No.:1,
   iii) a set of calibrators containing known scalar concentrations of the amino acid portion of Nox2 protein as sets forth in SEQ ID No.:1, or an amino acid sequence having at least 90%, preferably at least 95%, more preferably at least 98%, of identity with SEQ ID No.:1, wherein the known scalar concentrations are between 0 and 400 pg/ml,
   iv) instructions for use and,
   v) a buffer solution for diluting the biological sample.

11. The kit according to claim 10, wherein the set of calibrators comprises a blank and at least 4, preferably at least 7, calibrators.

12. The kit according to claim 10 or claim 11, wherein the known scalar concentrations of the amino acid portion of Nox2 protein as sets forth in SEQ ID No.:1, or an amino acid sequence having at least 90%, preferably at least 95%, more preferably at least 98%, of identity with SEQ ID No.:1, contained in the calibrators are between 0 and 200 pg/ml.

13. The kit according to any one of claims 10 to 12, in which the detection antibody is generated by the hybridoma PP7H10 deposited at the CBA-ICLC of Genoa with deposit number PD17002.

**Patentansprüche**

1. Diagnostisches Verfahren für den quantitativen Nachweis von Nox2-Protein in einer biologischen Probe, umfassend die folgenden Schritte:

   i) Normalisieren des Proteingehalts der biologischen Probe auf einen Wert von 1 $\mu$g Protein pro 100 $\mu$l Probe,
   ii) Inkontaktbringen der normalisierten biologischen Probe mit einem monoklonalen Anti-Nox2-Nachweisantikörper, wobei der Nachweisantikörper direkt mit einem Nachweismarker konjugiert ist, und
   iii) Nachweis der Bildung des Nox2-Protein-Nachweis-Antikörperkomplexes.

2. Verfahren nach Anspruch 1, wobei der Nachweisantikörper ein im Aminosäureanteil von Nox2 vorhandenes Epitop des Nox2-Proteins mit der in SEQ ID Nr. 1 angegebenen Sequenz erkennt.

3. Verfahren nach Anspruch 1 oder Anspruch 2, wobei der Schritt i) der Normalisierung des Proteingehalts der biologischen Probe durch Verdünnen der biologischen Probe mit einer Pufferlösung durchgeführt wird.

4. Verfahren nach einem der vorhergehenden Ansprüche, wobei der Nachweismarker gewählt ist aus radioisotopischen, enzymatischen, fluorimetrischen, biolumineszierenden, chemilumineszierenden Tracern.

5. Verfahren nach einem der vorhergehenden Ansprüche, wobei das Verfahren ferner die folgenden Schritte umfasst:

   iv) Erstellen einer Kalibrierungskurve unter Verwendung eines Satzes von Kalibratoren, die bekannte skalare Konzentrationen des Aminosäureanteils von Nox2-Protein wie in SEQ ID Nr. 1 angegeben oder einer Aminosäuresequenz mit mindestens 90 %, vorzugsweise mindestens 95 %, bevorzugter mindestens 98 % Identität mit SEQ ID Nr. 1 enthalten, und
   v) Bestimmen der Menge des Nox2-Proteins in der biologischen Probe durch Angabe des Ergebnisses des Schrittes iii) auf der in Schritt iv) erhaltenen Kalibrierungskurve.

6. Verfahren nach Anspruch 5, wobei die in den Kalibratoren enthaltenen bekannten skalaren Konzentrationen des Aminosäureanteils von Nox2-Protein wie in SEQ ID Nr. 1 angegeben oder einer Aminosäuresequenz mit mindestens 90 %, vorzugsweise mindestens 95 %, bevorzugter mindestens 98 % Identität mit SEQ ID Nr. 1 zwischen 0 und 400 pg/ml, vorzugsweise zwischen 0 und 200 pg/ml, liegen.

7. Verfahren nach Anspruch 5 oder Anspruch 6, wobei der Kalibratorsatz einen Leerwert und mindestens 4, vorzugsweise mindestens 7 Kalibratoren umfasst.

8. Verfahren nach einem der vorhergehenden Ansprüche, wobei der Nachweisantikörper von dem beim CBA-ICLC von Genua mit der Hinterlegungsnummer PD17002 hinterlegten Hybridom PP7H10 erzeugt wird.

9. Verfahren nach einem der vorhergehenden Ansprüche, wobei die biologische Probe gewählt ist aus Serum, Plasma, Zellüberstand.

10. Kit zur quantitativen Bestimmung des Nox2-Proteins in einer biologischen Probe, umfassend:

    i) eine feste Phase, die für die Durchführung eines diagnostischen Tests geeignet ist,
    ii) einen monoklonalen Anti-Nox2-Nachweisantikörper, der direkt an einen Nachweismarker konjugiert ist, wobei der Nachweisantikörper ein im Aminosäureanteil von Nox2 vorhandenes Epitop des Nox2-Proteins mit der in SEQ ID Nr. 1 angegebenen Sequenz erkennt,
    iii) einen Satz von Kalibratoren, die bekannte skalare Konzentrationen des Aminosäureanteils von Nox2-Protein wie in SEQ ID Nr. 1 angegeben oder einer Aminosäuresequenz mit mindestens 90 %, vorzugsweise mindestens 95 %, bevorzugter mindestens 98 % Identität mit SEQ ID Nr. 1 enthalten, wobei die skalaren Konzentrationen zwischen 0 und 400 pg/ml liegen,
    iv) eine Gebrauchsanweisung und
    v) eine Pufferlösung zum Verdünnen der biologischen Probe.

11. Kit nach Anspruch 10, wobei der Satz von Kalibratoren einen Leerwert und mindestens 4, vorzugsweise mindestens 7 Kalibratoren umfasst.

12. Kit nach Anspruch 10 oder Anspruch 11, wobei die in den Kalibratoren enthaltenen bekannten skalaren Konzentrationen des Aminosäureanteils von Nox2-Protein wie in SEQ ID Nr. 1 angegeben oder einer Aminosäuresequenz mit mindestens 90 %, vorzugsweise mindestens 95 %, bevorzugter mindestens 98 % Identität mit SEQ ID Nr. 1 zwischen 0 und 200 pg/ml liegen.

13. Kit nach einem der Ansprüche 10 bis 12, wobei der Nachweisantikörper von dem beim CBA-ICLC von Genua mit der Hinterlegungsnummer PD17002 hinterlegten Hybridom PP7H10 erzeugt wird.

**Revendications**

1. Méthode de diagnostic pour la détection quantitative de protéine Nox2 dans un échantillon biologique, comprenant les opérations suivantes :

    i) normalisation de la teneur en protéine de l'échantillon biologique à une valeur de 1 $\mu$g de protéine pour 100 $\mu$l d'échantillon,
    ii) mise en contact de l'échantillon biologique normalisé avec un anticorps de détection anti-Nox2 monoclonal, lequel anticorps de détection est directement conjugué à un marqueur de détection, et
    iii) détection de la formation du complexe de protéine Nox2-anticorps de détection.

2. Méthode selon la revendication 1, dans laquelle l'anticorps de détection reconnaît un épitope de la protéine Nox2 présent dans la partie d'acides aminés de Nox2 ayant la séquence telle que présentée dans la SEQ ID NO : 1.

3. Méthode selon la revendication 1 ou la revendication 2, dans laquelle l'opération de i) normalisation de la teneur en protéine de l'échantillon biologique est effectuée par dilution de l'échantillon biologique avec une solution tampon.

4. Méthode selon l'une quelconque des revendications précédentes, dans laquelle le marqueur de détection est choisi parmi les traceurs radioisotopiques, enzymatiques, fluorimétriques, bioluminescents, chimiluminescents.

5.  Méthode selon l'une quelconque des revendications précédentes, laquelle méthode comprend en outre les opérations suivantes :

    iv) élaboration d'une courbe d'étalonnage par utilisation d'un jeu de calibrateurs contenant des concentrations scalaires connues de la partie d'acides aminés de la protéine Nox2 telle que présentée dans la SEQ ID NO : 1, ou d'une séquence d'acides aminés ayant une identité d'au moins 90 %, de préférence d'au moins 95 %, mieux encore d'au moins 98 %, avec la SEQ ID NO : 1, et
    v) détermination de la quantité de la protéine Nox2 dans l'échantillon biologique par report du résultat de l'opération iii) sur la courbe d'étalonnage obtenue dans l'opération iv).

6.  Méthode selon la revendication 5, dans laquelle les concentrations scalaires connues de la partie d'acides aminés de la protéine Nox2, telle que présentée dans la SEQ ID NO : 1, ou d'une séquence d'acides aminés ayant une identité d'au moins 90 %, de préférence d'au moins 95 %, mieux encore d'au moins 98 %, avec la SEQ ID NO : 1, contenues dans les calibrateurs, sont comprises entre 0 et 400 pg/ml, de préférence entre 0 et 200 pg/ml.

7.  Méthode selon la revendication 5 ou la revendication 6, dans laquelle le jeu de calibrateurs comprend un témoin à blanc et au moins 4, de préférence 7, calibrateurs.

8.  Méthode selon l'une quelconque des revendications précédentes, dans laquelle l'anticorps de détection est généré par l'hybridome PP7H10 déposé au CBA-ICLC de Gênes avec le numéro de dépôt PD17002.

9.  Méthode selon l'une quelconque des revendications précédentes, dans laquelle l'échantillon biologique est choisi parmi un sérum, un plasma, un surnageant cellulaire.

10. Trousse pour la détermination quantitative de la protéine Nox2 dans un échantillon biologique, comprenant :

    i) une phase solide convenant pour effectuer un test diagnostique,
    ii) un anticorps de détection anti-Nox2 monoclonal directement conjugué à un marqueur de détection, lequel anticorps de détection reconnaît un épitope de la protéine Nox2 présent dans la partie d'acides aminés de Nox2 ayant la séquence telle que présentée dans la SEQ ID NO : 1,
    iii) un jeu de calibrateurs contenant des concentrations scalaires connues de la partie d'acides aminés de la protéine Nox2 telle que présentée dans la SEQ ID NO : 1, ou d'une séquence d'acides aminés ayant une identité d'au moins 90 %, de préférence d'au moins 95 %, mieux encore d'au moins 98 %, avec la SEQ ID NO : 1, lesquelles concentrations scalaires connues sont comprises entre 0 et 400 pg/ml,
    iv) des consignes d'utilisation, et
    v) une solution tampon pour diluer l'échantillon biologique.

11. Trousse selon la revendication 10, dans laquelle le jeu de calibrateurs comprend un témoin à blanc et au moins 4, de préférence 7, calibrateurs.

12. Trousse selon la revendication 10 ou la revendication 11, dans laquelle les concentrations scalaires connues de la partie d'acides aminés de la protéine Nox2 telle que présentée dans la SEQ ID NO : 1, ou d'une séquence d'acides aminés ayant une identité d'au moins 90 %, de préférence d'au moins 95 %, mieux encore d'au moins 98 %, avec la SEQ ID NO : 1, contenues dans les calibrateurs, sont comprises entre 0 et 200 pg/ml.

13. Trousse selon l'une quelconque des revendications 10 à 12, dans laquelle l'anticorps de détection est généré par l'hybridome PP7H10 déposé au CBA-ICLC de Gênes avec le numéro de dépôt PD17002.

**A**

$y=-4E-06x^2+0,0015x+0,0429$
$R^2$: 0.991

**B**

| A450 | sNOX2dp (pg/ml) |
|------|-----------------|
| 0,037 | 3,12 |
| 0,054 | 6,25 |
| 0,071 | 12,5 |
| 0,079 | 25 |
| 0,105 | 50 |
| 0,149 | 100 |
| 0,183 | 200 |

**C**

Figure 1

Method 1

Method 2

Figure 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6254547 B
- US 20120156704 A

### Non-patent literature cited in the description

- **BEDARD K. ; KRAUSE K.** *Physiol. Rev.,* 2007, vol. 87, 245-313
- **BRODSKY S.V. et al.** *Am. J. Physiol.,* 2004, vol. 286, H1910-1915
- **DWORAKOWSKY R. et al.** *Pharmacol. Reports,* 2008, vol. 60, 21-28
- **LIMING JIN ; BURNETT A.L.** *Asian J. Androl.,* 2008, vol. 10, 6-13
- **KITAS G.D. ; ERB N.** *Rheumatology,* 2003, vol. 42, 607-613
- **MARTINO F. et al.** *Pediatrics,* 2008, vol. 122, e648-e655
- **HALLIWELL B. ; WHITEMAN M.** *Br. J. Pharmacol.,* 2004, vol. 142, 231-255
- **DALE et al.** *Free Radic. Res.,* 2003, vol. 37, 815-821
- **CROSS A.R. ; ERICSON R.W. ; CURNUTE J.T.** *Biochem. J.,* 1999, vol. 341, 251-255
- **TEUFELHOFER O. et al.** *Toxicol. Sci.,* 2003, vol. 76, 376-383
- **PACLET et al.** *Biochemical Journal,* 2004, vol. 382 (3), 981-986
- **CAMPION et al.** *Biochemie,* 2007, vol. 89 (9), 1145-1158
- **COONEY et al.** *Journal of Neuroinflammation,* 2013, vol. 10 (1), 155
- **ALLRED et al.** *Journal of Lipid Research,* 2011, vol. 52 (2), 408-415
- **HEMPEN C.** *Anal. Bioanal. Chem.,* 2006, vol. 384, 572-583
- **WEBER D.** *Free Radical Research,* 2012, vol. 46, 276-285
- **BRADFORD MM.** *Analytical Biochemistry,* 1976, vol. 72, 248-254

SEQUENCE LISTING

<110> Università degli Studi di Roma "La Sapienza"

<120> Procedimento diagnostico per la determinazione della proteina Nox2

<130> BEP20519-CF

<160> 1

<170> PatentIn version 3.5

<210> 1
<211> 45
<212> PRT
<213> homo sapiens

<400> 1

```
Ala Glu Arg Ile Val Arg Gly Gln Thr Ala Glu Ser Leu Ala Val His
1               5                   10                  15

Asn Ile Thr Val Cys Glu Gln Lys Ile Ser Glu Trp Gly Lys Ile Lys
            20                  25                  30

Glu Cys Pro Ile Pro Gln Phe Ala Gly Asn Pro Pro Met
            35                  40                  45
```